# NEUE EUROPÄISCHE PATENTSCHRIFT

(11) **EP 0 895 069 B2**
(45) Veröffentlichungstag und Bekanntmachung des Hinweises auf die Entscheidung über den Einspruch: **22.12.2004**
(45) Hinweis auf die Patenterteilung: 14.11.2001
(21) Anmeldenummer: 98114090.8
(22) Anmeldetag: 28.07.1998
(51) Int. Cl.: G01G 17/00

(54) **Tablettentestgerät**
Testing device for tablets
Appareil de contrôle des comprimés

(30) Priorität: 30.07.1997 DE 19733436
(43) Veröffentlichungstag der Anmeldung: 03.02.1999
(60) Teilanmeldung: 99112469.4
(73) Patentinhaber: ERWEKA GmbH, D-63150 Heusenstamm (DE)
(72) Erfinder: Müller, Werner G., 60323 Frankfurt/Main (DE)
(74) Vertreter: Kirschner, Klaus Dieter, Dipl.-Phys.

(56) Entgegenhaltungen:
- EP-A- 0 685 714
- WO-A-90/01010
- DE-A- 4 241 985
- DE-U- 8 402 581
- FR-A- 2 142 655
- US-A- 4 393 717
- US-A- 4 472 960
- US-A- 4 884 463
- PATENT ABSTRACTS OF JAPAN vol. 095, no. 011, 26. Dezember 1995 & JP 07 206144 A (IKEGAMI TSUSHINKI CO LTD), 8. August 1995

## Beschreibung

Die Erfindung betrifft ein Tablettentestgerät zum Testen von Oblong-Tabletten oder ähnlich geformten Tabletten, insbesondere zum Wiegen, Vermessen und Bruchtesten derartiger Tabletten, in entsprechenden Teststationen.

Aus dem Deutschen Gebrauchsmuster 8 402 581 ist ein Tablettenprüfgerät mit einer Transporteinrichtung bekannt, bei dem die Tabletten von einer Zufuhreinrichtung auf eine Transporteinrichtung mit einem Rechen gelangen, der mehrere Gabeln aufweist, die die Tabletten auf einer Transportbahn weiterbewegen. Eine Bewegungseinrichtung für den Rechen transportiert den Rechen um eine Strecke in Richtung der Transportrichtung, hebt dann den Rechen an und führt ihn in angehobenem Zustand entgegen der Transportrichtung um eine entsprechende Strecke wieder zurück. Anschließend wird der Rechen abgesenkt und erneut um eine Transportstrecke weiterbewegt. Entlang der Bewegungsbahn des Rechens sind mehrere Teststationen, nämlich eine Waage, eine Teststation für die Dickenmessung und eine Teststation für den Bruchtest vorgesehen. Das bekannte Tablettentestgerät ist für runde oder kugelförmige Tabletten geeignet, weniger jedoch für sogenannte Oblong-Tabletten und ähnlich geformte Prüflinge, die eine im wesentlichen ovale Form in Draufsicht, einen fla-chen Steg an ihrem Umfang sowie eine gewölbte Ober- und Unterseite aufweisen können. Derartige Prüflinge bereiten auf dem bekannten Gerät Schwierigkeiten. Sie können in ver-schiedenen Teststationen, wie der Dickenmeßstation, der Durchmessermeßstation und der Station zur Bruchhärteprüfung nicht immer in der erforderlichen Weise ausgerichtet werden.

Aus der FR 2 142 655 A ist eine Einrichtung zum Wiegen und Sortieren von Tabletten bekannt, wobei die Tabletten zuerst gewogen werden und je nach dem Gewicht entweder verarbeitet oder verworfen werden.

Aus der US 4,393,717 A sind ein Verfahren und eine Vorrichtung zur automatischen Qualitätsprüfung von runden Tabletten oder Pillen bekannt. Die Tabletten werden von einer Produktionsmaschine in Vorratsbehälter abgegeben. Aus dem Tablettenstrom werden Muster zur Durchführung der Qualitätskontrolle abgezweigt, und die abgezweigten Tabletten werden in einer separaten Tablettenprüfeinrichtung getestet. Die runden Tabletten rollen von der Vorratseinrichtung über eine V-förmige Rinne in die Teststation, wo sie auf einem Teller einer Dickenmessung und einem Bruchtest unterzogen werden, wobei es wegen der Form der Tabletten nicht auf deren Orientierung auf dem Teller ankommt.

Aus der DE 42 41 985 Al ist eine Vorrichtung zum Prüfen von Tabletten bekannt, bei der einzelne Tabletten in kreisrunden Aufnahmen eines Drehtellers in eine Mittelachse zentriert und im Bereich einer Härteprüfstation mit einer Richtplatte so gedreht werden, dass ihre Längsachse sich in der radial verlaufenden Längsachse des Druckorgans einer Härteprüfeinrichtung befindet, um auch nicht kreissymmetrische Tabletten stets gleichen Prüfbedingungen im Härtetest zu unterwerfen. Für die Orientierung der länglichen Tabletten sind zusätzliche Zentrierungsmittel erforderlich.

Aus der "Bedienungsanleitung RQ100" vom 20. Juli 1993 der Fa. Kilian GmbH & Co. KG ist ein Gerät bekannt, mit dem Gewicht, Dicke und Härte von Tabletten gemessen werden kann. Die Tabletten werden von einem Fördertopf auf eine Waagschale gegeben und von dort über eine Tablettenrutsche auf einen Drehteller zur Dicken- und Härtemessung transportiert. Im Falle von Formtabletten wird eine spezielle Form des Drehtellers benutzt, um das Gerät auf das Messen von Formtabletten umzustellen, d.h. die Formtabletten werden durch die spezielle Ausgestaltung des Drehtellers so orientiert, daß eine reproduzierbare Dickenmessung möglich ist.

Der Erfindung liegt demgegenüber die Aufgabe zugrunde, ein Tablettentestgerät zum Testen von Oblong-Tabletten oder ähnlich geformten Tabletten in entsprechenden Teststätionen bereitzustellen, mit dem mehrere Test, beispielsweise das Wiegen, Vermessen und Bruchtesten in entsprechenden Teststationen durchgeführt werden kann, wobei die einzelnen Oblong-Tabletten oder ähnlich geformten Tabletten in den Meßstationen mit der erforderlichen Genauigkeit positioniert werden können.

Zur Lösung dieser Aufgaben ist das erfindungsgemäße Testgerät in der im Hauptanspruch angegebenen Weise gekennzeichnet.

Durch diese Ausgestaltung des erfindungsgemäßen Tablettentestgeräts kann sichergestellt werden, daß vergleichbare Ergebnisse beim Testen der Tabletten in den einzelnen Meßstationen erhalten werden. Die Dicke der Oblong-Tabletten oder ähnlichen Prüflingen ist definiert als die Dicke der Tablette senkrecht zu ihrer Längsachse und in der Mitte desselben. Daher wird die Dicke gemessen, indem die Tablette auf eine ihrer gewölbten Flächen gelegt und mit einem Taster gegen die gegenüberliegende gewölbte Fläche gefahren wird, um den Abstand zwischen dem Taster und der Auflagefläche zu messen. Daher muß die Tablette entsprechend positioniert sein, um diese Dickenmessung durchführen zu können. Die Länge einer Oblong-Tablette oder einer ähnlich geformten Tablette und die Bruchhärte werden entlang der Längsachse gemessen beziehungsweise überprüft, so daß auf die Längsachse in einer bestimmten Orientierung in der entsprechenden Teststation angeordnet sein muß. Diese Erfordemis-se können bei der erfindungsgemäßen Einrichtung leicht erfüllt werden, weil die Oblong-Tabletten oder ähnlichen Tabletten die richtige Orientierung haben, wenn sie auf der Transporteinrichtung ankommen.

Eine vorteilhafte Ausgestaltung der erfindungsgemäßen Einrichtung ist dadurch gekennzeichnet, daß die Zufuhreinrichtung eine in Transportrichtung geneigte Zufuhrrinne aufweist, in die die Tabletten insbesondere von einem Vibrations-Vereinzler eingespeist werden. In der Zufuhrrinne kann bereits eine gewisse Vororientierung der Tabletten erfolgen.

Eine weitere vorteilhafte Ausgestaltung des erfindungsgemäßen Tablettentestgeräts ist dadurch gekennzeichnet, daß zwischen der Waage und der Transporteinrichtung eine flache oder profilierte Übergaberinne vorgesehen ist, so daß in vorteilhafter Weise die Orientierung der Tabletten erhalten bleibt, die sie in der Waage erhalten haben.

Eine weitere vorteilhafte Ausgestaltung des erfindungsgemäßen Tablettentestgeräts ist dadurch gekennzeichnet, daß der Boden der Waagschale einen V-förmigen Querschnitt hat. Mit dieser Ausgestaltung der Rinne ist sichergestellt, daß die Prüflinge mit ihrer Längsachse im V-förmigen Querschnitt des Bodens der Waagschale ausgerichtet werden, wodurch eine optimale Orientierung der Oblong-Tablette erreicht wird.

Eine weitere vorteilhafte Ausgestaltung des erfindungsgemäßen Tablettentestgeräts ist dadurch gekennzeichnet, daß die Zufuhrrinne und/oder die Übergaberinne ebenfalls einen V-förmigen Querschnitt wie die Rinne der Waagschale aufweisen.

Eine weitere vorteilhafte Ausgestaltung des erfindungsgemäßen Tablettentestgeräts ist dadurch gekennzeichnet, daß zwischen der Stelle, an der die Oblong-Tabletten von der Waagschale kommend auf die Transporteinrichtung übergeben werden, und den weiteren Teststationen eine Schikane vorgesehen ist, der eventuell hochkant auf ihrem Steg stehende Oblong-Tabletten kippt, so daß sie auf ihre runde Seite fallen, und daß die Schikane als ein quer zur Transportrichtung vertikal vefahrbares Schikaneelement ausgebildet ist, das zur Bildung eines Stolpersteins über die Transportebene hinaus gefahren werden kann und zur Bildung einer Stolpernut unter die Tansportebene zurückgezogen werden kann.

Schließlich ist eine vorteilhafte Ausgestaltung des erfindungsgemäßen Tablettentestgerätes dadurch gekennzeichnet, daß die Transporteinrichtung eine Transportschiene und einen Rechen aufweist, der die Tabletten auf der Transportschiene vorwärts bewegt. Damit kann sichergestellt werden, daß die Orientierung der Oblong-Tablette, wenn sie von der Waage kommt, während des Weitertransports erhalten bleibt.

Ein Ausführungsbeispiel der Erfindung wird nun anhand der beiliegenden Zeichnung be-schrieben. Es zeigen:
- Figur 1: eine schematische Seitenansicht des oberen Teils eines Tablettentest . gerätes; und
- Figur 2: eine teilweise Draufsicht auf das Tablettentestgerät nach Figur 1; und
- Figur 3: eine Seitenansicht eines Details des Tablettentestgerätes nach Figur 1.

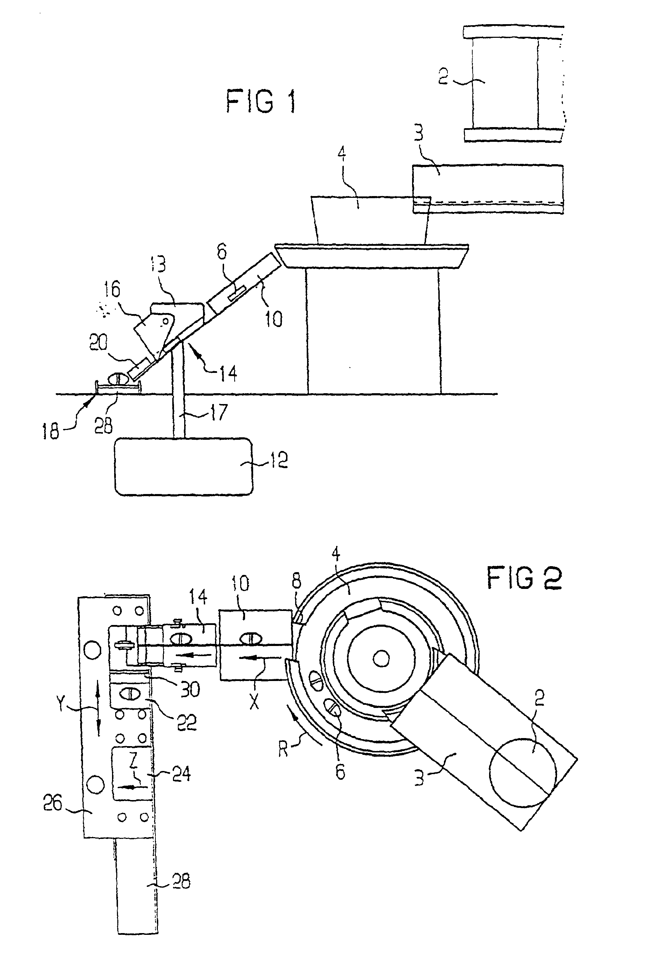

Wie aus den Figuren 1 und 2 zu ersehen ist, weist das Tablettentestgerät ein Magazin 2 beziehungsweise einen Vorratsbehälter für Prüflinge auf. Von dem Magazin 2 gelangen die Oblong-Tabletten in ein Vibrations-Vereinzlungssystem, das aus einer Längsförderrinne 3 und einem Rundförderer 4 besteht. Sowohl die Längsförderrinne 3 als auch der Rundförderer 4 können in bestimmten Geräteversionen entfallen. Von dem Rundförderer 4 werden die Tabletten 6 in Richtung des Pfeiles R (Figur 2) transportiert und über eine Abgabeöffnung 8 in eine Zufuhrrinne 10 abgegeben, die zu der Zufuhreinrichtung gehört und in Transportrichtung (Pfeil X, Figur 2) von der Auslaßöffnung 8 des Rundförderers 4 nach unten geneigt ist. Von der Zufuhrrinne 10 werden die Tabletten an eine Waage 12 übergeben, deren Waagschale 13 einen in Transportrichtung geneigten Boden 14 in Form einer Rinne aufweist. Die Waagschale 13 wird an ihrem unteren Ende von einer Klappe 16 verschlossen, die aus dem Weg der Tablette heraus bewegt werden kann, so daß diese die Waagschale 13 verlassen kann. Die Waagschale 13 mit dem Boden 14 und der Klappe 16 ist über einen Verbindungsstößel 17 mit der Waage 12 verbunden.

Zwischen der Waage 12 und einer Transporteinrichtung für die Oblong-Tabletten ist eine Übergaberinne 20 vorgesehen.

Die Waagschale 13 hat an ihrem Boden 14 eine V-förmigen Querschnitt. Die Zufuhrrinne 10 und/oder die Übergaberinne 20 können einen entsprechenden V-förmigen Querschnitt oder auch eine ebene Form aufweisen.

Die Transporteinrichtung 18 ist unterhalb der Waagschale 13 und unterhalb der Übergaberinne 20 vorgesehen, und die Oblong-Tabletten werden von der Waagschale 16 mit ihrer Längsachse parallel zur Längsrichtung der Rinne in dem Boden 14 der Waagschale abgelegt. DieTransporteinrichtung, die aus einer Transportschiene 28 und einem Rechen 26 besteht, transportiert die Tabletten in dieser Ausrichtung durch weitere Teststationen, insbesondere eine Teststation 22 für die Dickenmessung, in der ein Dickenmessungs-Sensor von oben an die Tablette herangefahren wird, und eine Teststation 24 in der der Durchmesser und die Bruchhärte der Tabletten gemessen wird. Der Doppelpfeil Y deutet an, daß der Rechen 26 sich über der Transportschiene 28 (Figur 1) hin und her bewegt, um die Oblong-Tabletten durch die Teststation zu transportieren. In dem Teil der Teststation 24, in dem der Bruchhärtetest durchgeführt wird, wirkt ein Bruchbacken (nicht gezeigt) in Richtung des Pfeiles Z auf die Oblong-Tabletten in deren Längsrichtung.

Zwischen der Stelle, an der die Oblong-Tabletten von der Waagschale 13 kommend auf die Transporteinrichtung 18 übergeben werden, und den weiteren Teststationen 22, 24 ist ein Stolperstein beziehungsweise ein Keil 30 in der Transporteinrichtung angeordnet, der eventuell hochkant auf ihrem Steg stehende Oblong-Tabletten kippt, so daß sie auf ihre runde Seite fallen.

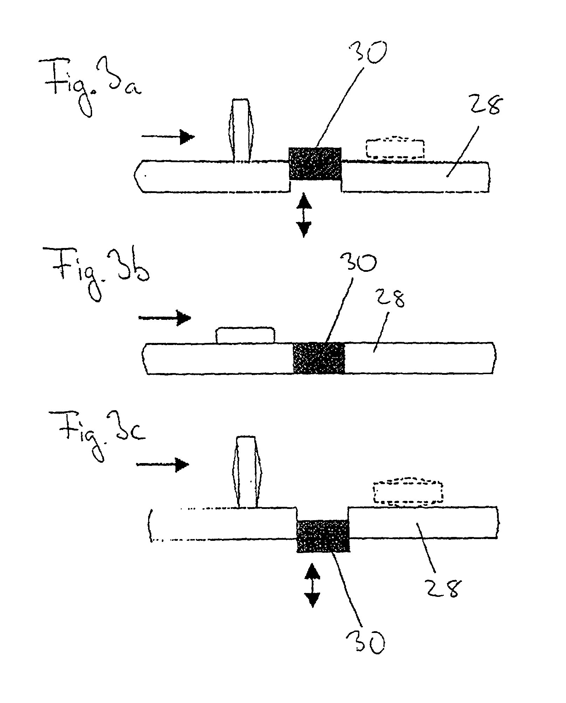

Figur 3 zeigt eine Ausrührungs form des Tablettentestgerätes nach Figur 1, bei dem die Stolpereinrichtung als vertikal verfahrbares Schikanenelement 30 ausgebildet ist, das quer zur Transportrichtung ausgerichtet ist und das zur Bildung eines Stolpersteines über die Transportebene hinausgefahren werden kann und zur Bildung einer Stolpernut unter die Transportebene zurückgezogen werden kann. Figur 3a zeigt das Schikanenelement 30 in einer über die Transportebene hinausgefahrenen Position zur Bildung eines Stolpersteines, Figur 3b zeigt das Schikanenelement 30 in einer Position bündig mit der Transporteinrichtung 28 und Figur 3c zeigt das Schikanenelement 30 in einer unter die Transportebene zurückgezogenen Position zur Bildung einer Stolpernut.

Aus der vorhergehenden Beschreibung ist ersichtlich, daß die Oblong-Tabletten oder ähnliche Tabletten in den Teststationen 22, 24, wo es auf die genaue Orientierung der Oblong-Tabletten ankommt, mit der richtigen Orientierung positioniert werden.

## Patentansprüche

1. Tablettentestgerät zum Testen von Oblong-Tabletten, insbesondere zum Wiegen, Vermessen und Bruchtesten derartiger Tabletten, in entsprechenden Teststationen, **gekennzeichnet durch** eine Zufuhreinrichtung (4, 10) zur Zufuhr einzelner Tabletten (6) an eine Waage (12), deren Waagschale (13) einen in Transportrichtung geneigten Boden (14) in Form einer Rinne zur Orientierung der Oblong-Tabletten mit ihrer Längsachse parallel zur Längsrichtung der Rinne in dem Boden (14) aufweist, und eine Transporteinrichtung (18) unterhalb der Waagschale (13), auf die die Tabletten von der Waageschale (13) mit ihrer Längsachse parallel zur Längsrichtung der Rinne abgelegt werden und die die Tabletten in dieser Ausrichtung **durch** weitere Teststationen (22, 24) transportiert.

2. Tablettentestgerät nach Anspruch 1, **dadurch gekennzeichnet, daß** die Zufuhreinrichtung eine zur Transporteinrichtung (18) geneigte Zufuhrrinne (10) aufweist, in die die Formlinge, insbesondere von einem Vibrations-Vereinzler, eingespeist werden.

3. Tablettentestgerät nach Anspruch 1 oder 2, **dadurch gekennzeichnet, daß** zwischen der Waage (12) und der Transporteinrichtung (18) eine Übergaberinne (20) vorgesehen ist.

4. Tablettentestgerät nach einem der vorhergehenden Ansprüche, **dadurch gekennzeichnet, daß** der Boden der Waagschale (13) einen V-förmigen Querschnitt hat.

5. Tablettentestgerät nach einem der vorhergehenden Ansprüche, **dadurch gekennzeichnet, daß** die Zufuhrrinne (10) und/oder die Übergaberinne (20) ebenfalls einen V-förmigen Querschnitt wie die Rinne der Waage (12) aufweist.

6. Tablettentestgerät nach Anspruch 1, **dadurch gekennzeichnet, daß** zwischen der Stelle, an der die Oblong-Tabletten von der Waagschale (13) kommend auf die Transporteinrichtung (18) übergeben werden, und den weiteren Teststationen (22, 24) eine Schikane vorgesehen ist, die eventuell hochkant auf ihrem Steg stehende Oblong-Tabletten kippt, so daß sie auf ihre runde Seite fallen, und daß die Schikane als ein quer zur Transportrichtung vertikal vefahrbares Schikaneelement (30) ausgebildet ist, das zur Bildung eines Stolpersteins über die Transportebene hinaus gefahren werden kann und zur Bildung einer Stolpemut unter die Tansportebene zurückgezogen werden kann.

7. Tablettentestgerät nach Anspruch 1, **dadurch gekennzeichnet, daß** die Transporteinrichtung (18) eine Transportschiene (28) und einen Rechen (26) aufweist, der die Tabletten auf der Transportschiene (28) vorwärts bewegt.

## Claims

1. An appliance for testing oblong tablets, especially for weighing, measuring and fracture testing of such tablets, in suitable testing stations, **characterized by** a feed means (4, 10) for the feed of individual tablets (6) to a weighing means (12), whose weighing pan (13) possesses a floor (14), inclined in the direction of the conveying, in the form of a trough for orienting the oblong tablet with its longitudinal axis in parallel to the longitudinal direction of the trough in the floor, and a conveying means (18) underneath the weighing pan (13), on which the tablets are deposited from the weighing pan (13) with their longitudinal axis parallel to the longitudinal direction of the trough and which conveys the tablets with such alignment through further testing stations (22, 24).

2. The tablet testing appliance as claimed in claim 1, **characterized in that** the feed means possesses a feed trough (10) inclined in relation to the conveying means (18), into which feed trough the tablets are fed, more particularly by a vibratory individualising means.

3. The tablet testing appliance as claimed in claim 1 or 2, **characterized in that** the weighing means (12) and the conveying means (18) have a transfer trough (20) arranged between them.

4. The tablet testing appliance as claimed in any of the preceding claims, **characterized in that** the floor of the weighing pan (13) possesses a V-shaped cross section.

5. The tablet testing appliance as claimed in any of the preceding claims, **characterized in that** the feed trough (10) and/or the transfer trough (20) also possess a V-shaped cross section as the weighing means (12).

6. The tablet testing appliance as claimed in claim 1, **characterized in that** between the point at which the oblong tablets coming from the weighing pan (13) are transferred to the conveying means (18), and the further testing stations (22, 24), a toppling means (30), is arranged in the conveying direction, which topples over any oblong tablets standing on their edges so that same fall onto their round side, and that the toppling means is built in the form of a toppling element (30) movable vertically transversely to the transport direction, which element any be moved beyond the transport plane to form a toppling stone and may be retracted below the transport plane to from the toppling recess.

7. The tablet testing appliance as claimed in claim 1, **characterized in that** the conveying means (18) possesses a conveying rail (28) and a rake (26), which moves the tablets on the conveying rail (28) forward.

## Revendications

1. Dispositif d'essai de comprimés pour tester les comprimés oblongs, en particulier pour peser, mesurer et tester la résistance à la rupture de ces comprimés, dans des postes d'essai correspondants, caractérisé en un mécanisme d'alimentation (4, 10) garantissant l'alimentation des comprimés (6), pièce par pièce, à un balance (12) dont le plateau (13) présente un fond (14) incliné dans la direction de transport en forme d'une gouttière pour l'orientation du comprimé oblong avec son axe longitudinal parallèle avec la direction longitudinale de la gouttière sur le fond (14), et en un mécanisme de transport (18) situé au-dessous du plateau (13), sur lequel les comprimés sont posés à partir du plateau (13), avec leurs axes longitudinaux paralléles au sens longitudinal de la gouttière, et qui transporte les comprimés en gardant leur orientation susmentionnée et les fait passer à travers d'autres postes d'essai (22, 24) à suivre.

2. Dispositif d'essai de comprimés selon la revendication 1, **caractérisé en ce que** le mécanisme d'alimentation présente une gouttière d'alimentation (10) inclinée vers le mécanisme de transport (18), dans laquelle les comprimés sont alimentés, notamment par un séparateur à vibrations.

3. Dispositif d'essai de comprimés selon les revendications 1 ou 2, **caractérisé en ce qu'**une gouttière de transfer (20) est prévue entre la balance (12) et le mécanisme de transport (18)

4. Dispositif d'essai de comprimés selon l'une quelconque des revendications précédentes, **caractérisé en ce que** le fond du plateau (13) présente une section transversale en forme d'un V.

5. Dispositif d'essai de comprimés selon la revendication 4, **caractérisé en ce que** la gouttière d'alimentation (10) et/ou la gouttière de transfer (20) présentent également une section transversale en forme d'un V comme celle de la gouttière de la balance (12).

6. Dispositif d'essai de comprimés selon la revendication 1, **caractérisé en ce qu'**une chicane (30) est prévue entre le lieu de transfer des comprimés oblongs venant du plateau (13) au mécanisme de transport (18) et les postes d'essai (22, 24) à suivre, cette chicane assurant que des éventuels comprimés oblongs se trouvant debout sur chant tombent sur leur face arrondie, et **en ce que** la chicane est réalisée comme élément de chicane (30) déplaçable verticalement et perpendiculairement à la direction de transport, pouvant être monté au-dessus du plan de transport pour former une chicane à butée et être descendu au-dessous du plan de transport pour former une chicane à rainure.

7. Dispositif d'essai de comprimés selon la revendication 1, **caractérisé en ce que** le mécanisme de transport (18) comprend une glissière (28) et un râteau (26) amenant les comprimés le long de la glissière (28).
